# EUROPEAN PATENT APPLICATION

(11) **EP 4 186 890 A1**
(43) Date of publication of application: **31.05.2023**
(21) Application number: 21383074.8
(22) Date of filing: 29.11.2021
(51) Int. Cl.: C07D 213/56, C07D 213/61

(54) **PREPARATION OF N-BENZYL-2-(5-BROMO-PYRIDIN-2-YL)-ACETAMIDE FOR THE SYNTHESIS OF TIRBANIBULIN**

(71) Applicant: Moehs Ibérica, S.L., 08191 Rubí-Barcelona (ES)
(72) Inventor: ARE, Celeste, 08191 Rubí, Barcelona (ES); BALLETTE, Roberto, 08191 Rubí, Barcelona (ES); DOBARRO RODRÍGUEZ, Alicia, 08191 Rubí, Barcelona (ES)
(74) Representative: ABG Intellectual Property Law, S.L.

(57) **Abstract**

The present invention relates to a process for the preparation of N-benzyl-2-(5-bromo-pyridin-2-yl)-acetamide, which is a suitable intermediate for the synthesis of tirbanibulin or a pharmaceutically acceptable salt thereof, as well as to a process for the preparation of tirbanibulin or a pharmaceutically acceptable salt thereof using said N-benzyl-2-(5-bromo-pyridin-2-yl)-acetamide intermediate.

## Description

### FIELD OF THE INVENTION

The present invention relates to a process for the preparation of N-benzyl-2-(5-bromo-pyridin-2-yl)-acetamide, which is an intermediate in the synthesis of tirbanibulin or a pharmaceutically acceptable salt thereof, as well as to a process for the preparation of tirbanibulin or a pharmaceutically acceptable salt thereof using said intermediate.

### BACKGROUND OF THE INVENTION

Tirbanibulin is a Src/pretubulin inhibitor developed by Athenex, Inc. for the treatment of Actinic Keratosis (AK).

Tirbanibulin is also known as N-benzyl-2-[5-[4-(2-morpholin-4-ylethoxy)phenyl]pyridin-2-yl]acetamide and has the chemical structure (V) depicted below.

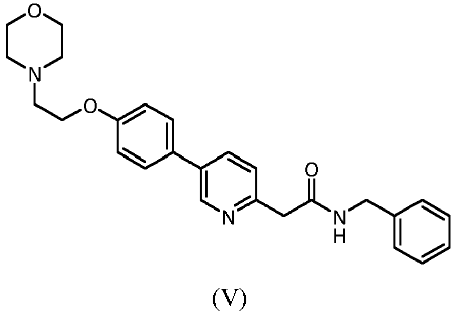

Tirbanibulin has been disclosed in patent document EP 1 836 169 B1. This document describes that tirbanibulin (V) is obtained by reaction of 4-{2-[4-(4,4,5,5-tetramethyl-[1,3,2]dioxaborolan-2-yl)-phenoxy]-ethyl}-morpholine with N-benzyl-2-(5-bromo-pyridin-2-yl)-acetamide in the presence of a Pd catalyst.

EP 1 836 169 B1 describes that tirbanibulin may be prepared through the following reaction:

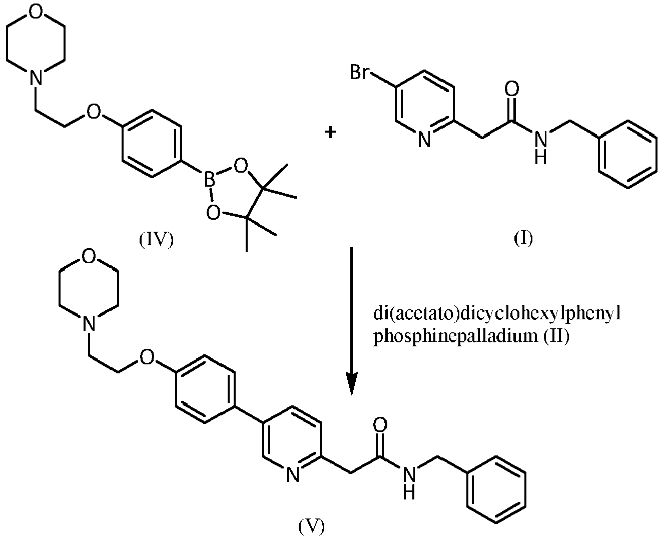

Thus, compound of formula (I) is known to be a useful intermediate for the synthesis of tirbanibulin.

The patent also discloses that compound (I) may be obtained by the following reaction: 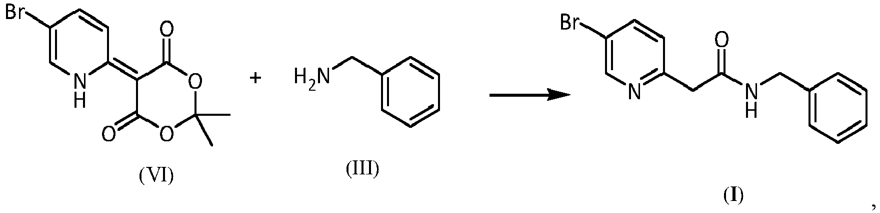 which is carried out in toluene brought to reflux under nitrogen for 18 hours producing (I) at a 96% yield. However, this process for preparing compound (I) is unsuitable for industrial production due to: a) the use of toluene which has been classified by the European Chemical Agency as suspected of damaging the unborn child and of organ damage through prolonged exposure (https://echa.europa.eu/information-on-chemicals/cl-inventory-database/-/discli/details/30426) and b) the need to work under nitrogen atmosphere.

An alternative preparation of compound (I) using simpler starting materials is described in Hangauer et al; J Med Chem, 2018 June 14th; 61(11): 4704-4719. Said paper describes the following process: 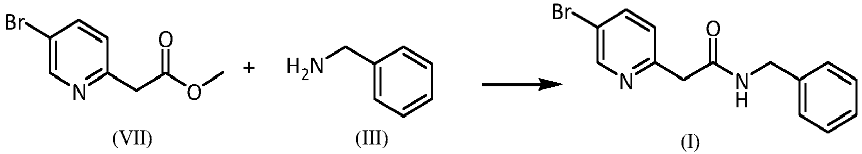 wherein the reaction takes place at 150°C using anhydrous anisole as a solvent during approximately 24 hours with a yield of 79%.

However, the process described above is suboptimal to be carried our industrially because: a) it employs high temperature (150°C) and b) it is difficult to effectively remove the residual reaction solvent present in the obtained product.

Thus, there is an unmet need to develop a process for the preparation of compound (I) which proceed with good yields and purities and is capable of been used industrially for the preparation of the compound.

### SUMMARY OF THE INVENTION

The inventors have surprisingly found a process for the production of compound of formula (I) in high yield using mild reaction conditions, which make the process suitable for industrial use. Said intermediate (I) can then be used for the synthesis of tirbanibulin or a pharmaceutically acceptable salt thereof.

Thus, in a first aspect, the present invention relates to a process for the preparation of N-benzyl-2-(5-bromo-pyridin-2-yl)-acetamide of formula (I) 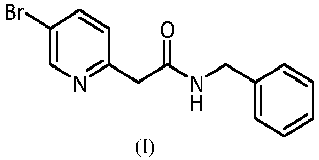 by reaction of (5-bromo-pyridin-2-yl)-acetic acid of formula (II) 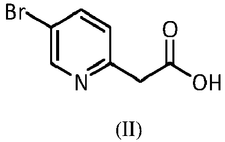 with benzylamine of formula (III)

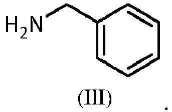

In a second aspect, the present invention relates to a process for the preparation of tirbanibulin of formula (V) 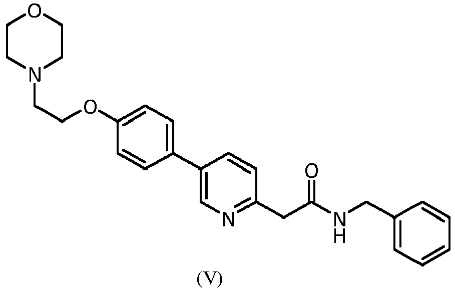 or a pharmaceutically acceptable salt thereof, said process comprising:
i) carrying out the process defined in the first aspect, thereby obtaining N-benzyl-2-(5-bromo-pyridin-2-yl)-acetamide of formula (I) 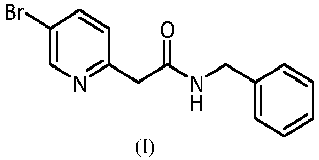
ii) reacting N-benzyl-2-(5-bromo-pyridin-2-yl)-acetamide of formula (I) obtained in step i) with 4- {2- [4-(4,4,5,5-Tetramethyl-[1,3,2]dioxaborolan-2-yl)-phenoxy]-ethyl}-morpholine of formula (IV) 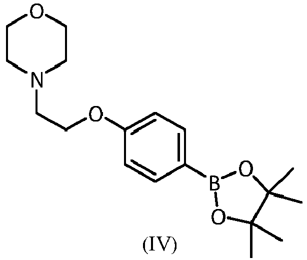 in the presence of a catalyst, thereby obtaining tirbanibulin of formula (V) 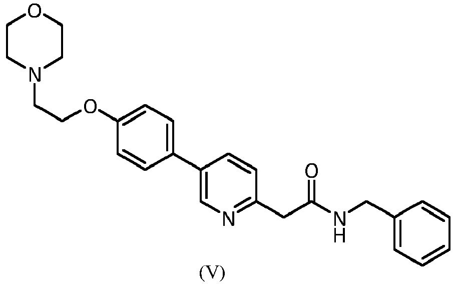
iii) optionally converting compound of formula (V) to a pharmaceutically acceptable salt thereof by reaction with a pharmaceutically acceptable acid.

### DESCRIPTION OF THE DRAWINGS

**Figure 1** shows the X-ray powder diffraction (XRPD) pattern of the compound of formula (I) obtained in Example 2.
**Figure 2** shows the X-ray powder diffraction (XRPD) pattern of the compound of formula (I) obtained in Example 3.

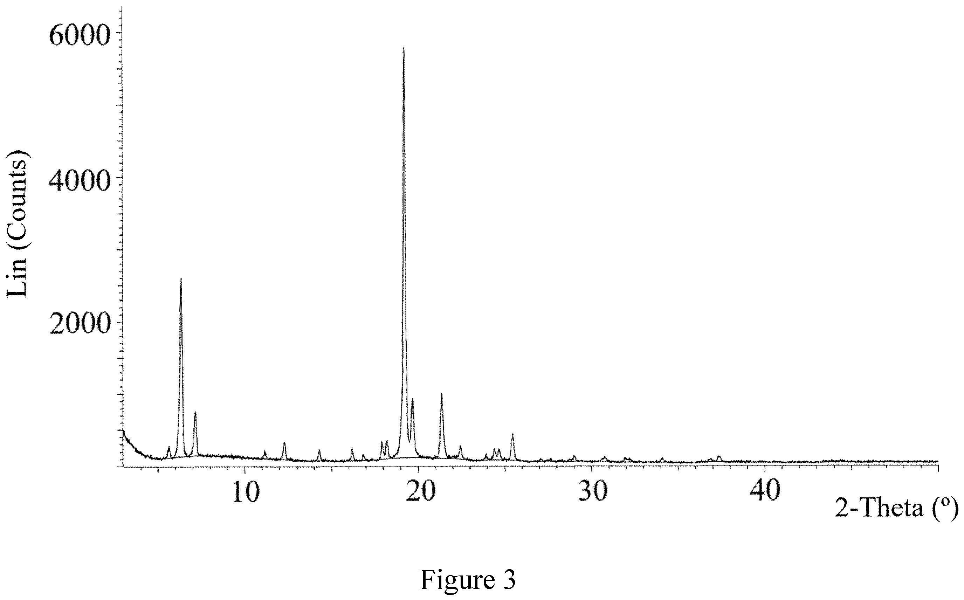

**Figure 3** shows the X-ray powder diffraction (XRPD) pattern of the compound of formula (V), i.e. tirbanibulin Form A obtained in Example 5.

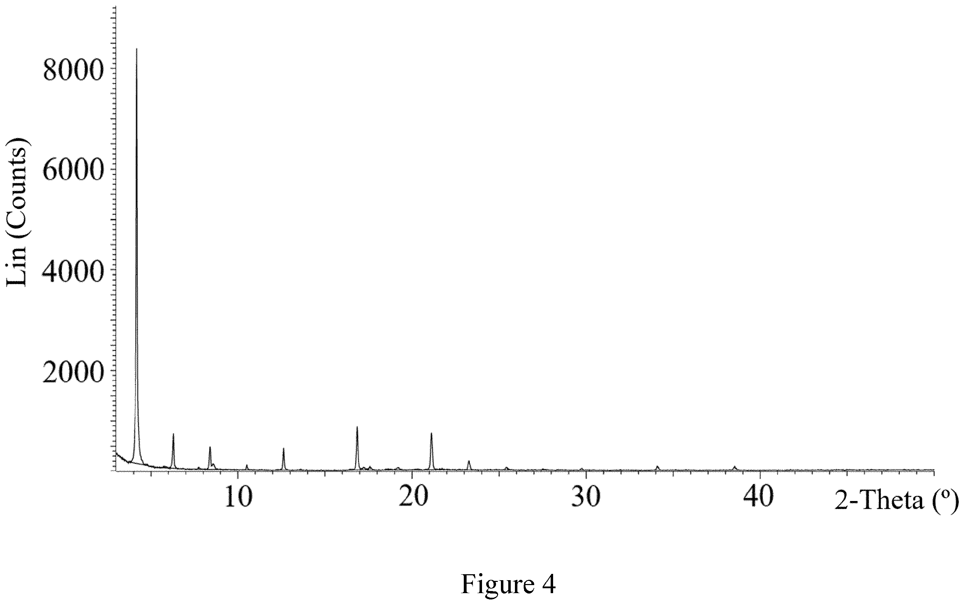

**Figure 4** shows the X-ray powder diffraction (XRPD) pattern of the compound of formula (V), i.e. tirbanibulin Form B obtained in Example 6.

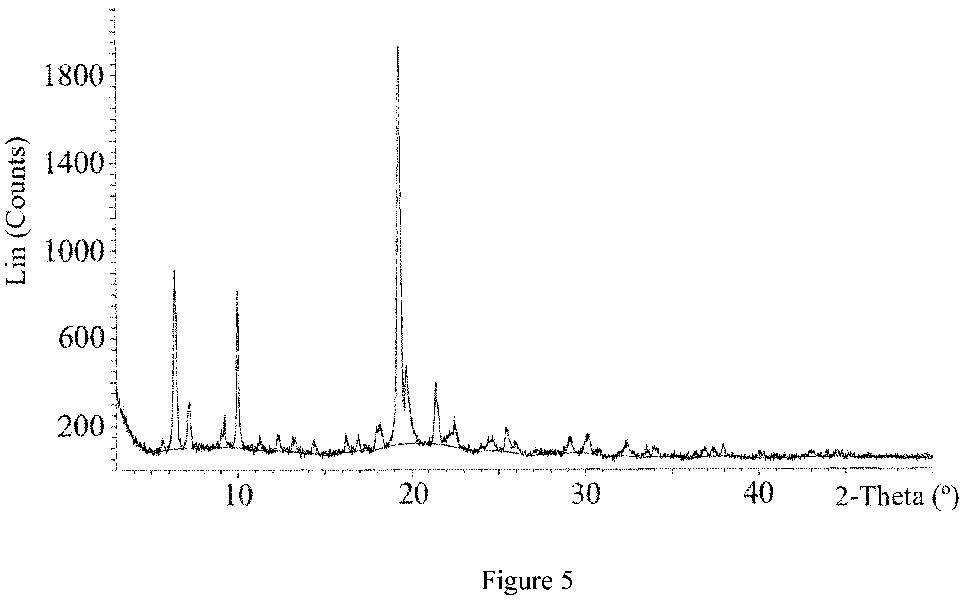

**Figure 5** shows the X-ray powder diffraction (XRPD) pattern of the compound of formula (V), i.e. tirbanibulin Form A obtained in Example 7.

### DESCRIPTION OF THE INVENTION

### Preparation of N-benzyl-2-(5-bromo-pyridin-2-yl)-acetamide (I)

In the first aspect, the present invention relates to a process for the preparation of N-benzyl-2-(5-bromo-pyridin-2-yl)-acetamide of formula (I) 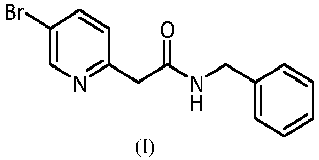 by reaction of (5-bromo-pyridin-2-yl)-acetic acid of formula (II) 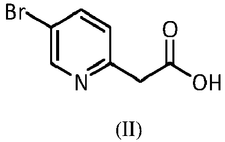 with benzylamine of formula (III)

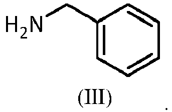

The above-mentioned reaction is preferably carried out in a solvent. The term "solvent" here refers to any liquid media capable of dissolving the compound of formula (II). In a preferred embodiment said solvent is selected from the group consisting of C₁-₆ alkyl acetates or cyclic ethers, in particular ethyl acetate, isopropyl acetate, tert-butyl acetate and 1,4-dioxane, more preferably ethyl acetate.

In a preferred embodiment, the reaction between the acid compound of formula (II) and the amine compound of formula (III) is carried out in the presence of a base and an activator of carboxylic acids for their reaction with amines.

The term "base" herein refers to a compound having a pKa (relative to water) of the corresponding conjugate acid of from 5 to less than 14. Examples of suitable bases are organic amines, such as alkylamines, including secondary and tertiary amines, wherein each alkyl moiety is independently selected from a C₁-C₆ alkyl group, preferably a C₁-C₄ alkyl group (such as triethylamine, diethylamine, diisopropylethylamine, tert-butylamine and the like), inorganic bases such as alkali metal or alkaline earth metal hydrogen carbonates (such as NaHCO₃), alkali metal or alkaline earth metal carbonates (such as Na₂CO₃, K₂CO₃), alkali metal hydroxides (such as sodium hydroxide and potassium hydroxide). Preferably, the base is an organic amine selected from the group consisting of triethylamine, diethylamine, diisoproylethylamine and tert-butylamine more preferably diisopropylethylamine. It is also possible to use the amine compound of formula (III) both as a reactant and as a base.

The expression "activator of carboxylic acids for their reaction with amines" is herein employed to designate any substance, which enhances the reactivity of carboxylic acids so that they react more efficiently with amines. Non-limiting examples of said activators are propylphosphonic acid anhydride (2,4,6-Tripropyl-1,3,5,2λ⁵,4λ⁵,6λ⁵-trioxatriphosphinane-2,4,6-trione), 1',1'-carbonyldiimida-zole, diisopropylcarbodiimide or the combination of diisopropylcarbodiimide and Oxyme Pure (Ethyl cyano(hydroxyimino)acetate), more preferably propylphosphonic acid anhydride (2,4,6-Tripropyl-1,3,5,2λ⁵,4λ⁵,6λ⁵-trioxatriphosphinane-2,4,6-trione) and 1',1'-carbonyldiimidazole and still more preferably propylphosphonic acid anhydride (2,4,6-Tripropyl-1,3,5,2λ⁵,4λ⁵,6λ⁵-trioxatriphosphinane-2,4,6-trione) or 1',1'-carbonyldiimidazole.

In a preferred embodiment, the amount of solvent employed is between 5 and 20 ml/g of compound of formula (II), preferably from 5 to 15 ml/g, more preferably from 7 to 12 mg/ml, still more preferably from 9.5 to 10.5 ml/g.

In a preferred embodiment, the amount of base employed is between 0.5 and 5 moles per mole of compound of formula (II), preferably from 1 to 3, most preferably from 1.5 to 2.5.

In a preferred embodiment, the amount of activator employed is between 1.0 and 3 moles per mole of compound of formula (II), preferably from 1 to 2, most preferably from 1.0 to 1.6.

In a preferred embodiment the reaction is carried out a temperature comprised between 0°C and 50 °C, more preferably between 0 and 40 °C, still more preferably between 0 and 30 °C and most preferably between 0 and 25 °C.

Once the above mentioned reaction has taken place, compound of formula (I) may be isolated by addition of water and maintenance of the resulting mixture a temperature between -10° C and 10°C, preferably between -5°C and 5°C, most preferably between - 2°C and 2°C, followed by separation of the resulting suspension by any suitable means, for example by filtration.

In a preferred embodiment, the compound of formula (I) is obtained in crystalline form, which is characterized by having an X-ray powder diffractogram measured with CuKa radiation with peaks at 5.9, 11.9, 19.1, 20.3, 22.6 and 25.9. In a embodiment the crystalline form is characterized by having an X-ray powder diffractogram measured with CuKa radiation with peaks at 5.9, 11.2, 11.9, 17.9, 19.1, 20.3, 21.5, 22.6, 23.9, 25.8, 27.4, 27.9, 30.0 °2θ±0.2 °θ.

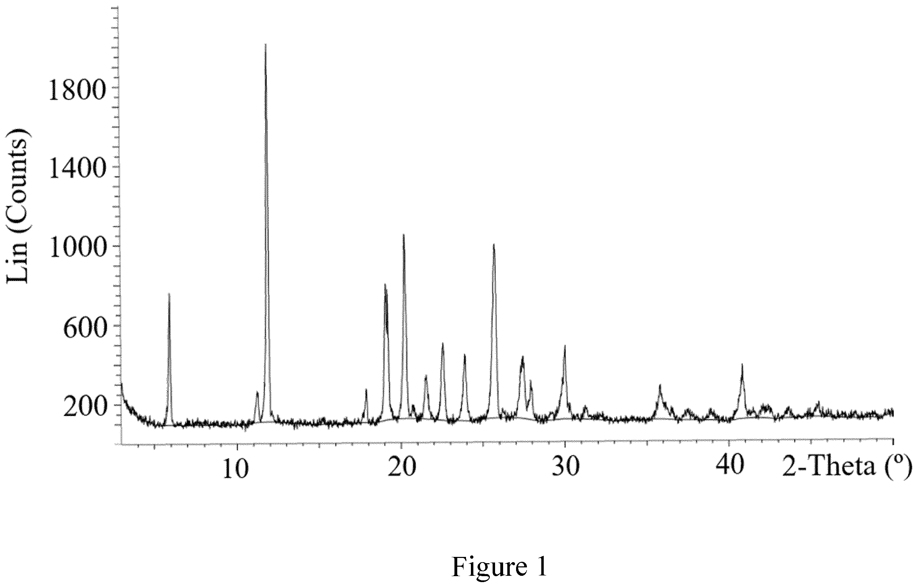

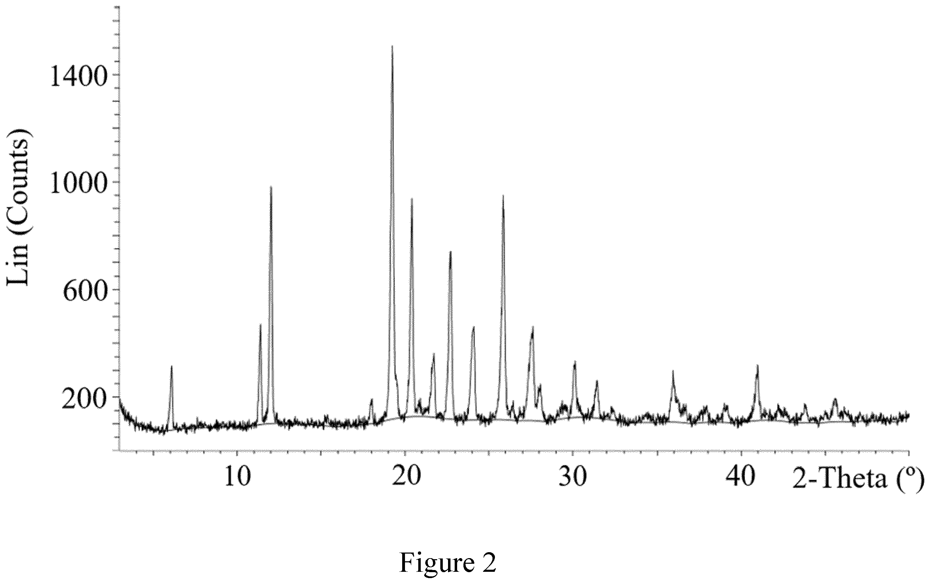

In a preferred embodiment the crystalline form is characterized by having an X-ray powder diffractogram measured with CuKa radiation essentially as that of Figures 1 or 2.

The X-ray diffractograms can be recorded using a powder diffraction system with a copper anode emitting CuKa radiation with a wavelength of 1.54060 Å, in particular, following the method described in the examples.

In a preferred embodiment, the compound of formula (I) is obtained having a differential scanning calorimetry (DSC) diagram comprising an endothermic peak with an onset temperature of about 157.6 °C ± 2 °C.

The differential scanning calorimetry diagram can be obtained as described in the examples.

The onset temperature refers to the temperature resulting from extrapolating the baseline before the start of the transition and the baseline during energy absorption (tangent to the curve). It can be calculated as defined in standard DIN ISO 11357-1:2016(E).

In a preferred embodiment compounds the molar ratio of compounds (III)/(II) is comprised between 0.8 and 2, preferably between 0.9 and 1.4, more preferably between 0.95 and 1.2.

### Preparation of tirbanibulin (V) or a pharmaceutically acceptable salt thereof

In a second aspect, the present invention relates to a process for the preparation of tirbanibulin of formula (V) 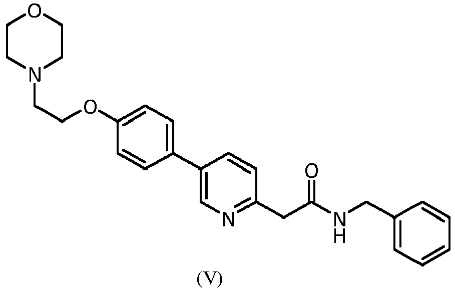 or a pharmaceutically acceptable salt thereof, said process comprising:
i) carrying out the process defined in the first aspect, thereby obtaining N-benzyl-2-(5-bromo-pyridin-2-yl)-acetamide of formula (I) 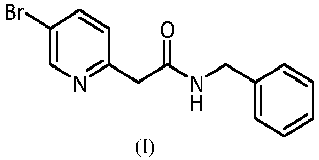
ii) reacting N-benzyl-2-(5-bromo-pyridin-2-yl)-acetamide of formula (I) obtained in step i) with 4- {2- [4-(4,4,5,5-Tetramethyl-[1,3,2]dioxaborolan-2-yl)-phenoxy]-ethyl}-morpholine of formula (IV) 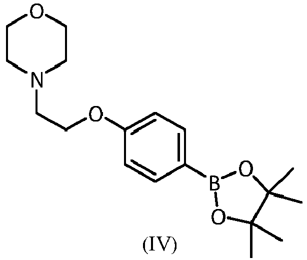 in the presence of a catalyst, thereby obtaining tirbanibulin of formula (V) 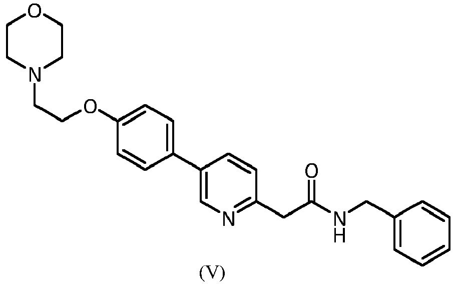
iii) optionally converting compound of formula (V) to a pharmaceutically acceptable salt thereof by reaction with a pharmaceutically acceptable acid.

The reaction of step ii) is preferably carried out in a solvent. The term "solvent" here refers to any liquid media capable of dissolving the compounds of formulae (I) and (IV). In a preferred embodiment said solvent is selected from the group consisting of C₁₋₄dialkyl ketones, C₁₋₆alkylalcohols, cyclic ethers, water or mixtures thereof. Examples of suitable solvents are acetone, methylethylketone, methanol, ethanol, tert-butanol, n-butanol, tetrahydrofuran, 1,4-dioxane, water and mixtures thereof. Preferably the solvent is acetone, methanol, 1,4-dioxane, water or mixtures thereof. More preferably the solvent is a mixture of acetone and water, a mixture of 1,4-dioxane and water or a mixture of methanol and water. The most preferably solvent is a mixture of methanol and water.

In a preferred embodiment, the reaction between the compound of formula (I) and the compound of formula (IV) is carried out in the presence of a base and a catalyst.

The term "base" herein refers to a compound having a pKa (relative to water) of the corresponding conjugate acid of from 5 to less than 14. Examples of suitable bases are inorganic bases such as alkali metal or alkaline earth metal hydrogen carbonates (such as NaHCO₃), alkali metal or alkaline earth metal carbonates (such as Na₂CO₃, K₂CO₃), alkali metal hydroxides (such as sodium hydroxide and potassium hydroxide) or alkali metal phosphates (such as K₃PO₄). More preferably, the base is Na₂CO₃.

The term "catalyst" is herein employed to designate complexes of palladium or complexes of nickel. Non-limiting examples of said catalysts are [1,1'-Bis(di-tert-butylphosphino)ferrocene]dichloro palladium (II), complex of [1,1'-Bis(diphenylphosphino)ferrocene]dichloropalladium(II) with dichloromethane, 9-[Dicyclohexyl[2',4',6'-tris(propan-2-yl)-[1,1'-biphenyl]-2-yl]-λ-4-phosphanyl}-O-methanesulfonyl-8-methyl-8-λ-4-aza- 9-palladium (II) (Xphos Pd G4) or complex of acetate of palladium (II) with tri-tert-butylphosphonium tetrafluoroborate. In a preferred embodiment the catalyst is [1,1'-Bis(di-tert-butylphosphino)ferrocene]dichloro palladium (II) or its complex with dichloromethane .

In a preferred embodiment the molar ratio of compounds (IV)/(I) is comprised between 0.5 and 3, preferably between 1 and 2, more preferably between 1.1 and 1.6, most preferably between 1.1 and 1.3.

In a preferred embodiment, the amount of solvent employed is between 10 and 30 ml/g of compound of formula (I), preferably from 15 to 25 ml/g, more preferably from 18 to 22 mg/ml, still more preferably from 19.5 to 20.5 ml/g.

In a preferred embodiment, the amount of base employed is between 0.5 and 5 moles per mole of compound of formula (I), preferably from 2 to 4, most preferably from 2.5 to 3.5.

In a preferred embodiment, the amount of catalyst employed is between 0.005 and 0.050 moles per mole of compound of formula (I), preferably from 0.010 to 0.030, most preferably from 0.010 to 0.020.

In a preferred embodiment, the reaction is carried out a temperature comprised between 0°C and 80 °C, more preferably between 20 and 70 °C, still more preferably between 30 and 60 °C and most preferably between 50 and 60 °C.

Once the above-mentioned reaction has taken place, compound of formula (V) may be isolated by evaporation of the solvent to obtain a residue, which is then mixed with methanol and warmed at a temperature between 30 and 60 °C, preferably between 40 and 50°C. The mixture is then filtered to obtain a solution (A). Alternatively, compound of formula (V) may be isolated by filtering the mixture of reaction to obtain a solution (B). To the above mentioned solutions (A) or (B) water is added maintaining the temperature between 30 and 60 °C, preferably between 40 and 55°C. After cooling the mixture is filtered obtaining tirbanibulin of formula (V) .

When it is desired to obtain pharmaceutically acceptable salts of the compound of formula (V), i.e. tirbanibulin said compound is caused to react with the corresponding pharmaceutically acceptable acid, preferably in an excess of said acid.

The process of the invention provides tirbanibulin or a pharmaceutically acceptable salt thereof in high yield and chemical purity.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

Further, the term "pharmaceutically acceptable salt" refers to any salt, which, upon administration to the recipient is capable of providing (directly or indirectly) a compound as described herein. Due to the nature of the chemical groups in tirbanibulin, the pharmaceutically acceptable salt thereof may be acid addition salts, and they can be synthesized from the parent compound, which contains a basic moiety by conventional chemical methods. Generally, such salts are, for example, prepared by reacting the free base form of this compound with a stoichiometric amount of the appropriate acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, monohydrochloride, dihydrochloride, hydrobromide, hydroiodide, sulphate, nitrate, phosphate, and organic acid addition salts such as, for example, acetate, benzoate, maleate, fumarate, citrate, lactate, glycolate, gluconate, oxalate, pamoate, succinate, tartrate, malate, mandelate, malonate, adipate, acorbate, benzenosulphonate, methanesulphonate, p-toluenesulphonate, naphthalenosulphonate, and the like.

Preferably, the pharmaceutically acceptable salt of tirbanibulin is methanesulphonate, monohydrochloride or dihydrochloride salt.

The following examples represent specific embodiments of the present invention. They do not intend to limit in any way the scope of the invention defined in the present description.

### Examples

### XPRD Difractograms

XRPD analysis were performed using a model X-ray powder diffractometer BRUKER D2 PHASER equipped with a Copper anode. The radiation used is CuKa with a wavelength of 1.54060 Å. Scan parameters: 3-50 degrees 2θ, continuous scan, ratio: 5.6 degrees / minute.

### DSC analysis

DSC analysis was performed in a Mettler Toledo 822e apparatus with STARe SW15 software. Parameters: heating range of 30 to 300 °C with a ramp of 10 °C/min and N₂ flow of 50 ml/min. The measurement is taken with a perforated closed capsule.

### Chromatographic methods

### Method for determining purity by Ultra High Performance Liquid Chromatography (UHPLC)

The purity of compounds obtained in examples 1 to 6 was measured using a Acquity BEHC18 100 x 2.1 mm, 1.7µm column at 40 °C. The chromatograph was equipped with UV detector working at 270 nm for compounds obtained by means of method disclosed in examples 1, 2, 3, 5, 6, 7 and 8, and 240 nm for compound obtained by means of method disclosed in example 4. The flow rate was 0.3 mL/min. Test samples were prepared by dissolving the appropriate amount of sample (1 mg/mL) in 50:50 acetonitrile/water and the volume injected of sample was 1 µL.

The mobile phase A was obtained solving 1 mL of phosphoric acid 85% in 200 mL of water. The mobile phase B was acetonitrile. The mobile phase C was water. The chromatograph was programmed as follow:

| *t* | *%A* | *%B* | %C |
|---|---|---|---|
| 0.0 | 5 | 15 | 80 |
| 0.5 | 5 | 15 | 80 |
| 4.0 | 5 | 95 | 0 |
| 5.0 | 5 | 95 | 0 |
| 5.8 | 5 | 15 | 80 |
| 8 | 5 | 15 | 80 |

### Example 1. Synthesis of 5-(bromo-pyridin-2-yl)acetic acid (II)

100 gr (0.35 mol) of 5-bromo-2-iodopyridine, 344.3 g (1.05 mol) of cesium carbonate, 6.7 g (0.035 mol) of copper (I) iodide and 8.67 g (0.07 mol) of picolinic acid were solved in 120 mL of 1,4-dioxane. A previously prepared solution of 67.7 g (0.42 mmol) of ethyl malonate in 120 mL of 1,4-dioxane were slowly added while keeping the temperature between 25 and 28 °C. The resulting mixture was heated to about 70 °C and stirred for 18 hours at about 70 °C.

500 mL of ethyl acetate and 500 mL of water were added and the resulting mixture was stirred for 1 hour at temperature about 25 °C. The mixture was filtered over Celite and the cake was washed with 200 mL of ethyl acetate. The organic layer was separated and the aqueous phase washed with 250 mL of ethyl acetate. The combined organic extracts were concentrated in vacuo to afford a yellow oil comprising 2-(5-bromo-pyridin-2-yl)malonic acid diethyl ester.

The oil was solved in 1 L of methanol and 700 mL (1.4 mol) of a 2M aqueous solution of sodium hydroxide was slowly added. The mixture of reaction was stirred for 4 hours at temperature about 25 °C.

Once the maintenance was finished, the solvent was removed by vacuum distillation and the residue was dissolved by adding 500 mL of methyl-*tert*-butyl ether and 500 mL of water. The organic phase was discarded and the resulting aqueous phase was acidified by addition of concentrate acid chloride until pH of about 2. The resulting suspension was filtered to afford a yellowish wet solid that was dried with vacuum at about 40 °C to obtain 63.6 g (83.6% yield) of 5-(bromo-pyridin-2-yl)acetic acid (II) (UHPLC purity: 97.90%).

### Example 2. Synthesis of N-benzyl-2-(5-bromopyridin-2-yl)acetamide (I)

45 gr (0.208 mol) of 5-(bromo-2-pyridin-2-yl)acetic acid (II) were mixed with 450 mL of ethyl acetate. 53.76 g (0.416 mol) of diisopropylethylamine were added to obtain a solution. 24.51 g (0.220 mol) of benzylamine (III) were added to obtain a white suspension and finally 173.6 mL (0.291 mol) of a 50% solution of propylphosphonic anhidre (T3P^{®}) in ethyl acetate was slowly added. All the additions were performed at a temperature between 20 and 25 °C, except the later addition that was performed at a temperature of between 15 and 20 °C. The obtained mixture was stirred for 7 hours at temperature of about 25 °C.

Once the maintenance was finished, 225 mL of water were slowly added to the obtained solution at a temperature of between 20 and 25 °C observing the presence of a solid. The suspension was slowly cooled to a temperature of about 0 °C and maintained for about 1 hour at temperature of about 0 °C. The resulting suspension was filtered to afford a wet solid that was washed with 100 mL of water and further was dried with vacuum at about 45 °C to obtain 59.8 g (94.0% yield) of a white solid corresponding to N-benzyl-2-(5-bromopyridin-2-yl)acetamide (I) (UHPLC purity: 99.87%).

### Example 3. Synthesis of N-benzyl-2-(5-bromopyridin-2-yl)acetamide (I)

5 gr (0.023 mol) of 5-(bromo-2-pyridin-2-yl)acetic acid (II) and 4.5 g (0.028 mol) of 1',1'-carbonyldiimidazole were mixed with 50 mL of ethyl acetate. The mixture was stirred for about 30 minutes at a temperature of about 20 °C. 4.96 g (0.046 mol) of benzylamine (III) were added at a temperature of about 0 °C to obtain a yellow suspension. The obtained mixture was stirred for 2 hours at temperature of about 0 °C.

Once the maintenance was finished, 50 mL of water were slowly added to the mixture at a temperature of between 0 and 5 °C. The suspension was slowly stirred for about 2 hours at temperature of about 0 °C. The resulting suspension was filtered to afford a wet solid that was washed with 25 mL of water and further was dried with vacuum at about 45 °C to obtain 6.3 g (89.5% yield) of a white solid corresponding to N-benzyl-2-(5-bromopyridin-2-yl)acetamide (I) (UHPLC purity: 99.94%).

### Example 4. Synthesis of 4-(2-(4-(4.4,5,5-tetramethyl(1,3,2]diaxaborolan-2-yl)-phenoxy)ethyl)morpholine (IV)

522 mL of DMF were added to 52.2 g (0.237 mol) of 4-hydroxybenzeneboronic acid pinacol ester (VIII), 72.12 g (0.521 mol) of potassium carbonate and 1.969 g (0.012 mol) of potassium iodide. 52.94 gr (0.285 mol) of the hydrochloride salt of 4-(2-Chloroethyl)morpholine HCl (IX) were further added at temperature about 25 °C. The obtained mixture was heated at a temperature of between 70 and 75 °C and maintained during 18 hours under stirring at said temperature.

Once the maintenance was finished, the mixture was cooled at a temperature of about 20 °C and 522 mL of water and 522 mL of tert-butyl methyl ether were added. The organic layer was separated and the aqueous layer was washed with one fraction of 261 mL of tert-butyl methyl ether. The combined organic layer was washed with brine (261 mL). The solvent was removed by vacuum distillation and the obtained residue was dissolved by adding 260 mL of isopropyl alcohol and heating the mixture at reflux temperature. The obtained solution was slowly cooled at a temperature of about 30 °C appearing the presence of a solid. The emulsion is stirring at said temperature for about 1 hour and further slowly cooled at a temperature of about 5 °C. The mixture is stirring at said temperature for about 2 hours. The resulting suspension was filtered to afford a solid that was dried with vacuum at about 40 °C to obtain 60.9 g (77.0% yield) of a white solid corresponding to 4-(2-(4-(4.4,5,5-tetramethyl[1,3,2]diaxaborolan-2-yl)-phenoxy)ethyl)morpholine (IV) (UHPLC purity: 99.80%).

### Example 5. Synthesis of N-benzyl-2-(5-4-(2-morpholinoethoxy)phenyl)pyridin-2-yl)acetamide (Tirbanibulin) (V)

26.72 g (0.088 mol) of N-benzyl-2-(5-bromopyridin-2-yl)acetamide (I), obtained by means the procedure disclosed in example 2, and 35.0 g (0.105 mol) of 4-(2-(4-(4.4,5,5-tetramethyl[1,3,2]diaxaborolan-2-yl)-phenoxy)ethyl)morpholine, obtained by means the procedure disclosed in example 4, were dissolved in a mixture of 267 mL of acetone and 267 mL of water. 27.85 g (0.263 mol) of sodium carbonate were added to the solution at temperature of about 25 °C. Finally, 1.14 g (0.00175 mol) of [1,1'-Bis(di-tert-butylphosphino)ferrocene]dichloro palladium (II) were added to the solution at temperature of about 25 °C. The mixture of reaction was heated at a temperature of about 55 °C and maintained for 5 hours at said temperature.

Once the maintenance was finished, the solvent was removed by vacuum distillation and the obtained residue was mixed with 566 mL of methanol and the mixture was warmed at a temperature of about 45 °C. The mixture was filtered to obtain a solution and 1132 mL of water were slowly added maintaining the temperature of the mixture between 45 and 50 °C. The mixture was slowly cooled at a temperature of between 20 and 25 °C and maintained for 2 hours at said temperature. The resulting suspension was filtered to afford a solid that was washed with a mixture of 19 mL of methanol and 38 mL of water dried with vacuum at about 45 °C to obtain 33.66 g **(89.1% yield)** of a white crystalline solid corresponding to N-benzyl-2-(5-4-(2-morpholin-4-ylethoxy)phenyl)pyridin-2-yl)acetamide (V) (UHPLC purity: 99.78%). The crystalline form obtained by means of the disclosed example has been identified by means of its corresponding X-ray diffraction pattern (XRPD). It corresponds to the crystalline form named as Form A as it was disclosed in the International patent application with publication number WO 2019/051147 A.

### Example 6. Synthesis of N-benzyl-2-(5-4-(2-morpholinoethoxy)phenyl)pyridin-2-yl)acetamide (V) (Tirbanibulin)

1 g (3.27 mmol) of N-benzyl-2-(5-bromopyridin-2-yl)acetamide (I), obtained by means the procedure disclosed in example 2, and 1.6 g (4.91 mmol) of 4-(2-(4-(4.4,5,5-tetramethyl[1,3,2]diaxaborolan-2-yl)-phenoxy)ethyl)morpholine (IV), obtained by means the procedure disclosed in example 4, were dissolved in 20 mL of a mixture of 1,4-dioxane/water (5:1). 1 g (9.81 mmol) of sodium carbonate were added to the mixture at temperature of about 25 °C. Finally, 0.106 g (0.16 mmol) of [1,1'-Bis(di-tert-butylphosphino)ferrocene]dichloro palladium (II) were added to the mixture at temperature of about 25 °C. The mixture of reaction was heated at a temperature of about 90 °C and maintained for 8 hours at said temperature.

Once the maintenance was finished, the suspension was filtered over Celite, the organic layer was separated and the solvent was finally removed by vacuum distillation. The obtained residue was mixed with 10 mL of isopropyl alcohol and the mixture was warmed at a temperature of about 50 °C. The obtained solution was slowly cooled at a temperature of between 20 and 25 °C and maintained overnight at said temperature. The resulting suspension was filtered to afford a solid that was dried with vacuum at about 40 °C to obtain 1.24 g **(88.0% yield)** of a white crystalline solid corresponding to N-benzyl-2-(5-4-(2-morpholin-4ylethoxy)phenyl)pyridin-2-yl)acetamide (V) (UHPLC purity: 99.01%). The crystalline form obtained by means of the disclosed example has been identified by means of its corresponding X-ray diffraction pattern (XRPD). It corresponds to the crystalline form named as Form B as it was disclosed in the International patent application with publication number WO 2019/051147 A.

### Example 7. Synthesis of N-benzyl-2-(5-4-(2-morpholinoethoxy)phenyl)pyridin-2-yl)acetamide (Tirbanibulin) (V)

1 g (3.27 mmol) of N-benzyl-2-(5-bromopyridin-2-yl)acetamide (I), obtained by means the procedure disclosed in example 2, and 1.6 g (4.91 mmol) of 4-(2-(4-(4.4,5,5-tetramethyl[1,3,2]diaxaborolan-2-yl)-phenoxy)ethyl)morpholine (IV), obtained by means the procedure disclosed in example 4, were dissolved in 20 mL of a mixture of methanol/water (1:1). 1 g (9.81 mmol) of sodium carbonate were added to the mixture at temperature of about 25 °C. Finally, 0.106 g (0.16 mmol) of [1,1'-Bis(di-tert-butylphosphino)ferrocene]dichloro palladium (II) were added to the mixture at temperature of about 25 °C. The mixture of reaction was heated at a temperature of about 80 °C and maintained for 3 hours at said temperature.

Once the maintenance was finished, the mixture was filtered over Celite and 10 mL of water were slowly added maintaining the temperature about 50 °C. The mixture was slowly cooled at a temperature of between 20 and 25 °C and maintained overnight at said temperature. The resulting suspension was filtered to afford a solid that was washed with a mixture of 1 mL of methanol and 2 mL of water dried with vacuum at about 45 °C to obtain 1.21 g **(85.5% yield)** of a white crystalline solid corresponding to N-benzyl-2-(5-4-(2-morpholin-4-ylethoxy)phenyl)pyridin-2-yl)acetamide (V) (UHPLC purity: 99.10%). The crystalline form obtained by means of the disclosed example has been identified by means of its corresponding X-ray diffraction pattern (XRPD). It corresponds to the crystalline form named as Form A as it was disclosed in the International patent application with publication number WO 2019/051147 A.

### Example 8. Synthesis of N-benzyl-2-(5-4-(2-morpholin-4-ylethoxy)phenyl)pyridin-2-yl)acetamide (Tirbanibulin) (V)

1 g (3.27 mmol mol) of N-benzyl-2-(5-bromopyridin-2-yl)acetamide (I), obtained by means the procedure disclosed in example 2, and 1.3 g (3.93 mmol) of 4-(2-(4-(4.4,5,5-tetramethyl[1,3,2]diaxaborolan-2-yl)-phenoxy)ethyl)morpholine, obtained by means the procedure disclosed in example 4, were dissolved in a mixture of 20 mL of methanol/water (5:1). 1 g (9.81 mmol) of sodium carbonate were added to the solution at temperature of about 25 °C. Finally, 0.021 mg (0.0327 mol) of [1,1'-Bis(di-tert-butylphosphino)ferrocene]dichloro palladium (II) were added to the solution at temperature of about 25 °C. The mixture of reaction was heated at a temperature of about 75 °C and maintained for 6 hours at said temperature.

Once the maintenance was finished, the mixture was filtered over Celite and 30 mL of water were slowly added maintaining the temperature about 50 °C. The mixture was slowly cooled at a temperature of between 20 and 25 °C and maintained overnight at said temperature. The resulting suspension was filtered to afford a solid that was washed with a mixture of 1 mL of methanol and 2 mL of water dried with vacuum at about 45 °C to obtain 1.25 g (88.4% yield) of a white crystalline solid corresponding to N-benzyl-2-(5-4-(2-morpholin-4-ylethoxy)phenyl)pyridin-2-yl)acetamide (V) (UHPLC purity: 99.21%).

## Claims

1. Process for the preparation of of N-benzyl-2-(5-bromo-pyridin-2-yl)-acetamide of formula (I) 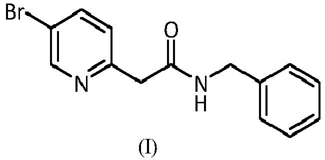 by reaction of (5-bromo-pyridin-2-yl)-acetic acid of formula (II) 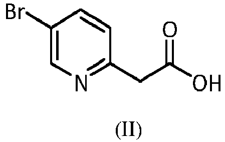 with benzylamine of formula (III)

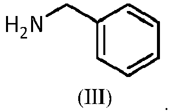

2. Process according to claim 1, wherein the reaction is carried out in a solvent, preferably selected from the group consisting of C₁₋₆ alkyl acetates or cyclic ethers, preferably selected from the group consisting of ethyl acetate, isopropyl acetate, tert-butyl acetate and 1,4-dioxane, more preferably ethyl acetate.

3. Process according to any of the preceding claims, wherein the reaction is carried out in the presence of a base and an activator of carboxylic acids.

4. Process according to any of the preceding claims, wherein the base is selected from the group consisting of organic amines, including secondary and tertiary alkyl amines, wherein each alkyl moiety is independently selected from a C₁-C₆ alkyl group, preferably a C₁-C₄ alkyl group inorganic bases selected from the group consisting of alkali metal or alkaline earth metal hydrogen carbonates, alkali metal or alkaline earth metal carbonates and alkali metal hydroxides, preferably selected from the group consisting of triethylamine, diethylamine, diisoproylethylamine and tert-butylamine, more preferably diisopropylethylamine or wherein the base is the compound of formula (III) or a mixture of the bases recited above and the compound of formula (III).

5. Process according to any of the preceding claims, wherein the activator of carboxylic acids is selected from the group consisting of propylphosphonic acid anhydride (2,4,6-Tripropyl-1,3,5,2λ,⁵4λ,⁵ 6λ⁵-trioxatriphosphinane-2,4,6-trione), 1',1'-carbonyldiimida-zole, diisopropylcarbodiimide or the combination of diisopropylcarbodiimide and Oxyme Pure (Ethyl cyano(hydroxyimino)acetate), more preferably selected from the group consisting of propylphosphonic acid anhydride (2,4,6-Tripropyl-1,3,5,2λ⁵,4λ⁵,6λ⁵-trioxatriphosphinane-2,4,6-trione) and 1',1'-carbonyldiimidazole, more preferably propylphosphonic acid anhydride (2,4,6-Tripropyl-1,3,5,2λ⁵,4λ⁵,6λ⁵-trioxatriphosphinane-2,4,6-trione) or 1',1'-carbonyldiimidazole.

6. Process according to any of the preceding claims, wherein the amount of solvent employed is between 5 and 20 ml/g of compound of formula (II), preferably from 5 to 15 ml/g, more preferably from 7 to 12 mg/ml, still more preferably from 9.5 to 10.5 ml/g.

7. Process according to any of the preceding claims, wherein the amount of base employed is between 0.5 and 5 moles per mole of compound of formula (II), preferably from 1 to 3, most preferably from 1.5 to 2.5 and the amount of activator employed is between 1.0 and 3 moles per mole of compound of formula (II), preferably from 1 to 2, most preferably from 1.0 to 1.6.

8. Process according any of the preceding claims, wherein the reaction is carried out a temperature comprised between 0°C and 50 °C, more preferably between 0 and 40 °C, still more preferably between 0 and 30 °C and most preferably between 0 and 25 °C.

9. Process for the preparation of tirbanibulin of formula (V) 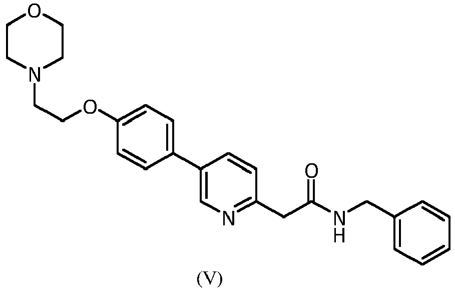 or a pharmaceutically acceptable salt thereof, said process comprising:
i) carrying out the process defined in any one of claims 1 to 8, thereby obtaining N-benzyl-2-(5-bromo-pyridin-2-yl)-acetamide of formula (I) 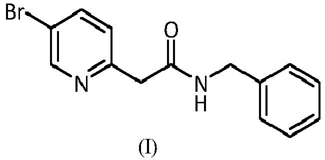
ii) reacting N-benzyl-2-(5-bromo-pyridin-2-yl)-acetamide of formula (I) obtained in step i) with 4- {2- [4-(4,4,5,5-Tetramethyl-[1,3,2]dioxaborolan-2-yl)-phenoxy]-ethyl}-morpholine of formula (IV) 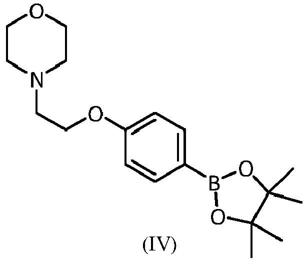 in the presence of a catalyst, thereby obtaining tirbanibulin of formula (V) 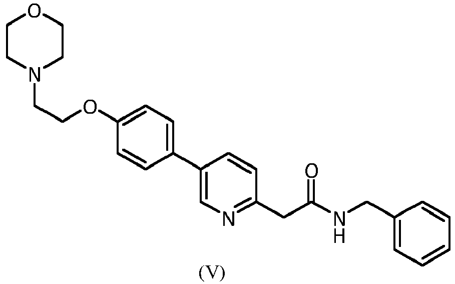
iii) optionally converting compound of formula (V) to a pharmaceutically acceptable salt thereof by reaction with a pharmaceutically acceptable acid.

10. Process according to claim 9, wherein step ii) is carried out in a solvent, preferably selected from the group consisting of C₁₋₄-dialkyl ketones, C₁₋₆alkylalcohols, cyclic ethers, water or mixtures thereof, preferably selected from the group consisting of acetone, methylethylketone, methanol, ethanol, tert-butanol, n-butanol, tetrahydrofuran, 1,4-dioxane, water and mixtures thereof, more preferably selected from the group consisting of acetone, methanol, 1,4-dioxane, water or mixtures thereof even more preferably selected from the group consisting of acetone, water, a mixture of 1,4-dioxane and water or a mixture of methanol and water, most preferably a mixture of methanol and water.

11. Process according to any of claims 9 to 10, wherein the reaction is carried out in the presence of a base, preferably selected from the group consisting of inorganic bases, preferably selected from the group consisting of alkali metal or alkaline earth metal hydrogen carbonates, alkali metal or alkaline earth metal carbonates, alkali metal hydroxides and alkali metal phosphates, more preferably selected from the group consisting of NaHCO₃, Na₂CO₃, K₂CO₃, K₃PO₄, more preferably Na₂CO₃ and a catalyst, preferably selected from the group consisting of [1,1'-Bis(di-tert-butylphosphino)ferrocene]dichloro palladium (II), a complex of [1,1'-Bis(diphenylphosphino)ferrocene]dichloropalladium(II) with dichloromethane, 9-[Dicyclohexyl[2',4',6'-tris(propan-2-yl)-[1,1'-biphenyl]-2-yl]-λ-4-phosphanyl}-O-methanesulfonyl-8-methyl-8-λ-4-aza- 9-palladium (II) and a complex of acetate of palladium (II) with tri-tert-butylphosphonium tetrafluoroborate.

12. Process according any one of claims 9 to 11, wherein the molar ratio of compounds (IV)/(I) is comprised between 0.5 and 3, preferably between 1 and 2, more preferably between 1.1 and 1.6, most preferably between 1.1 and 1.3.

13. Process according to any one of claims 9 to 11, wherein the amount of solvent employed is between 10 and 30 ml/g of compound of formula (I), preferably from 15 to 25 ml/g, more preferably from 18 to 22 mg/ml, still more preferably from 19.5 to 20.5 ml/g.

14. Process according to any one of claims 9 to 13, wherein the amount of base employed is between 0.5 and 5 moles per mole of compound of formula (I), preferably from 2 to 4, most preferably from 2.5 to 3.5.

15. Process according to any one of claims 9 to 14, wherein the amount of catalyst employed is between 0.005 and 0.050 moles per mole of compound of formula (I), preferably from 0.010 to 0.030, most preferably from 0.010 to 0.020
